# EUROPÄISCHE PATENTSCHRIFT

(11) **EP 1 080 076 B1**
(45) Veröffentlichungstag und Bekanntmachung des Hinweises auf die Patenterteilung: **19.11.2003**
(21) Anmeldenummer: 99924901.4
(22) Anmeldetag: 07.05.1999
(51) Int. Cl.: C07D 221/26

(54) **VERBESSERTES VERFAHREN ZUR HERSTELLUNG VON PHARMAZEUTISCH WERTVOLLEN NORBENZOMORPHANDERIVATEN**
IMPROVED METHOD FOR PREPARING PHARMACEUTICALLY VALUABLE NORBENZOMORPHANE DERIVATIVES
PROCEDE AMELIORE DE PRODUCTION DE DERIVES DE NORBENZOMORPHANE PRECIEUX SUR LE PLAN PHARMACEUTIQUE

(30) Priorität: 20.05.1998 DE 19822822
(43) Veröffentlichungstag der Anmeldung: 07.03.2001
(73) Patentinhaber: Boehringer Ingelheim Pharma GmbH & Co.KG, 55218 Ingelheim am Rhein (DE)
(72) Erfinder: GRAUERT, Matthias, D-55218 Ingelheim (DE); BALTES, Hanfried, D-55597 Wöllstein (DE); SCHNAUBELT, Jürgen, D-88400 Biberach (DE)
(74) Vertreter: Laudien, Dieter, Dr.
(86) Internationale Anmeldenummer: EP9903142
(87) Internationale Veröffentlichungsnummer: WO99059976

(56) Entgegenhaltungen:
- DE-A- 19 528 472

## Beschreibung

Die vorliegende Erfindung betrifft ein neues Verfahren zur Herstellung von Norbenzomorphanderivaten der allgemeinen Formel **1** (in den Abbildungen 1a und 1b sind die entsprechenden Stereoisomere dargestellt, im Text wird lediglich auf die Herstellung des R-Enantiomeren eingegangen - die S-Enantiomeren sind in analoger Weise herstellbar): 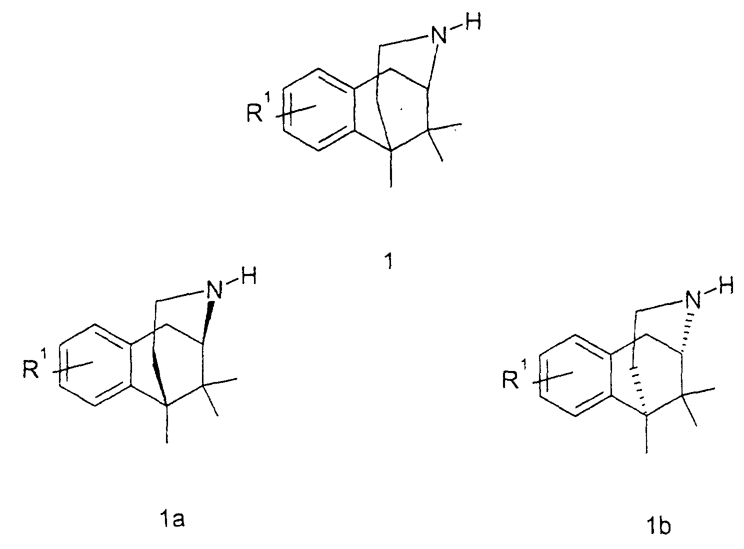 worin
R¹ = H, C₁-C₈-Alkyl, C₁-C₈-Alkoxy, Hydroxy oder Halogen
bedeuten kann.

Soweit nicht im einzelnen abweichende Angaben gemacht werden, werden die allgemeinen Definitionen im folgenden Sinn gebraucht:
C₁-C₈-Alkyl steht im allgemeinen für einen verzweigten oder unverzweigten Kohlenwasserstoffrest mit 1 bis 8 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
   Methyl, Ethyl, Propyl, 1-Methylethyl (Isopropyl), Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylproypyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2,-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 3 Kohlenstoffatomen, wie Methyl; Ethyl, Propyl, Isopropyl.
C₁-C₈-Alkoxy steht im allgemeinen für einen verzweigten oder unverzweigten über ein Suaerstoff gebundenen Kohlenwasserstoffrest mit 1 bis 8 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
   Methoxy, Ethoxy, Propoxy, 1-Methylethyl (Isopropyl), Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylproypoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2,-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Bevorzugt sind - sofern nicht anders angegeben - Niederalkoxyreste mit 1 bis 3 Kohlenstoffatomen, wie Methoxy Ethoxy, Propoxy, Isopropoxy.

Halogen bedeutet im Sinne der vorliegenden Verbindung Fluor, Chlor, Brom, Jod worunter Fluor und Chlor als Substituenten bevorzugt sind. Als Anionen in Aluminiumverbindungen werden Brom und Chlor bevorzugt - letzteres besonders.

Das Verfahren kann zur Synthese der racemischen Verbindungen sowie zur Synthese der entsprechenden enantiomerenreinen Verbindungen genutzt werden. Gegenüber dem in der Deutschen Offenlegungsschrift 195 28 472 beschriebenen Verfahren weist das erfindungsgemäße Verfahren den Vorteil auf, daß man zwei Stufen - nämlich das Einführung sowie das nachfolgende Entfernen der N-Formyl-Schutzgruppe - einspart. Im Falle des 4'-Methoxy substituierten Norbenzomorphans (R¹= 4'-OMe), das ein wertvolles Zwischenprodukt für pharmazeutisch wirksame Norbenzomorphanderivate darstellt, erhält man darüber hinaus deutlich bessere Ausbeuten der gewünschten Verbindung.

Im eingangs genannten Stand der Technik wird ein Verfahren beschrieben, in dem entsprechenden 4-Methylen-piperidinderivate **2** nach dem Einführen einer N-Formylschutzgruppe - **3** - zu den entsprechenden Benzomorphanderivaten **4** cyclisiert werden. Um zu den entsprechenden Norbenzomorphanen **5** zu gelangen muß die Formylschutzgruppe in einem weiteren Schritt jedoch wieder abgespalten werden.

Anschließend kann - falls gewünscht - der Substituent R² auf an sich bekannte Weise zu R¹ gemäß der Zielverbindung **1** modifiziert werden. So kann, falls R² die Bedeutung einer Alkoxygruppe hat - wie z.B. Methoxy, Ethoxy, n-Propoxy oder iso-Propoxy - auf dem Wege einer Etherspaltung - z.B. durch Umsetzung mit einer Halogenwasserstoffsäure wie HBr - die entsprechende Hydroxyverbindung (R¹ = OH) generiert werden.

Überraschenderweise wurde nun gefunden, daß mit dem erfindungsgemäßen Verfahren nunmehr auf die Einführung einer Formylschutzgruppe verzichtet werden kann. Erfindungsgemäß läßt sich das Piperidinderivat **2** in der protonierten Form direkt mit AlCl₃ zum Benzomorphanderivat **5** cyclisieren. Die Synthese ist in Schema 1 für die entsprechenden 1R-Enantiomere dargestellt. Sie kann jedoch auch mit den entsprechenden 1S-Enantiomeren oder mit den racemischen
Ausgangsverbindungen analog durchgeführt werden.

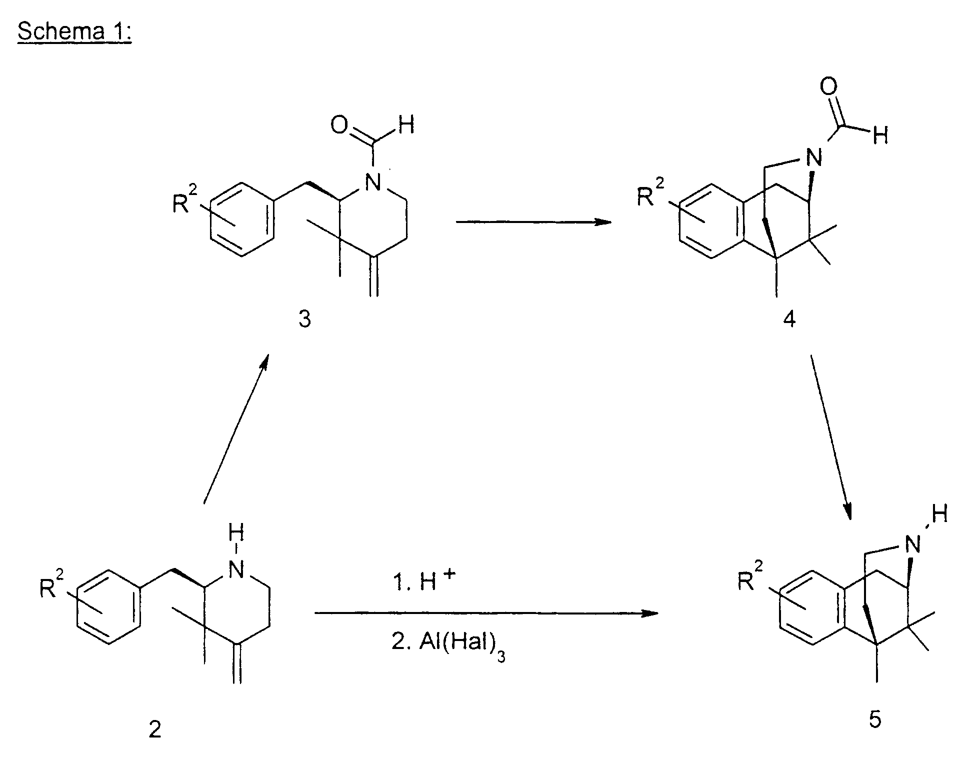

So erhält man nach dem im Stand der Technik beschriebenen Verfahren im Falle des 2-(2-Methoxyphenyl)methyl-3,3-dimethyl-4-methylen-piperidins **2a** (R²= 2-OMe) das gewünschte Benzomorphanderivat nur zu 20%. Das neue Verfahren hingegen liefert das gewünschte Benzomorphanderivat vom Typ **5** - im Beispiel mit R²=OCH³ - in über 80% isolierter Ausbeute.

Variationen der Versuchsbedingungen (Tabelle 1) zeigen, daß für eine erfogreiche Cyclisierung das 4-Methylen-piperidin **2** zunächst lediglich in ein Salz überführt werden muß, da die Cyclisierung der freien Base überwiegend Zersetzungsprodukte unbekannter Natur liefert.

Das erfindungsgemäße Verfahren wird zweckmäßigerweise in einem Reaktionsmedium durchgeführt. Als Reaktionsmedien eignen sich dabei insbesondere halogenierte aliphatische oder aromatische Kohlenwasserstoffe oder aber auch Säureamide, worunter mono- oder polychloriere Alkane mit 1 bis 3 C-Atomen oder chlorierte Benzol(-Derivate) oder Säureamide von Carbonsäuren mit 1 bis 3 C-Atomen in der Carboxyleinheit besonders bevorzugt sind. Ganz besonders bevorzugt sind Dichlormethan (Methylenchlorid), 1,2-Dichlorethan, Chlorbenzol und Dimethylacetamid. Es könne aber auch Mischungen der genannten Lösungsmittel eingesetzt werden.

Die Reaktionstemperatur ist für die erfindungsgemäße Reaktion in weiten Bereichen unkritisch. Sie richtet sich im erster Linie nach der Reaktivität der Reaktionsteilnehmer, während die Obergrenze durch den Siedepunkt des Lösungsmittel gesetzt wird - sofern die Reaktion nicht im Autoklaven durchgeführt wird. Somit kann die erfindungsgemäße Reaktion in Abhängigkeit vom eingesetzten Lösungsmittel in einem Temperaturintervall von 0 bis 150 °C durchgeführt werden. Bevorzugt wird ein Bereich von 20 bis 100 °C, worin ein Intervall von 40 bis 70 °C besonders bevorzugt wird.

Die eingesetzte Menge an Aluminium(III)halogenid- vorzugsweise Aluminumtribromid und besonders bevorzugt Aluminiumtrichlorid - ist ebenfalls in weiten Bereichen variierbar. Sie liegt typischerweise in einem Intervall von 2 bis 12 Äquivalenten Aluminiumchlorid bezogen auf das Edukt. Besonders bevorzugt wird ein Verhältnis in einem Bereich von 3 bis 10 Äquivalenten, worin ein Verhältnis in einem Intervall von 3 bis 5 Äquivalenten besonders bevorzugt wird.

Die zum Einsatz gelangende Salzform ist bezüglich der vorteilhaften Durchführbarkeit der erfindungsgemäßen Reaktion ebenfalls nicht kritisch. Bevorzugt werden die Salze des Piperidinderivates vom Typ 2 mit anorganischen Säuren - insbesondere mit Mineralsäuren eingesetzt. Bevorzugt werden die- neutralen - Salze mit Halogenwasserstoffsäuren oder Schwefelsäure. Neben neutralen Sulfaten (in Tabelle 1 mit "SU1" abgekürzt) kommen besonders bevorzugt Hydrochloride (Cl) oder Hydrobromide (Br) zum Einsatz.

Die vorstehend beschriebene Erfindung wird daneben durch das in den nachfolgenden Beispielen beschriebene Verfahren erläutert. Verschiedenartige, andere Ausgestaltungen des erfindungsgemäßen Verfahrens werden für den Fachmann aus der vorliegenden Beschreibung ersichtlich. Es wird jedoch ausdrücklich darauf hingewiesen, daß die Beispiele und die Beschreibung lediglich zur Erläuterung vorgesehen und nicht als Einschränkung der Erfindung anzusehen sind.

### Beispiele

### Beispiel 1: (-)-4'-Methoxy-5,9,9-trimethyl-6,7-benzomorphan-tartrat ((-)-5aTA)

4,9 g (20 mmol) (+)-2-(2-Methoxyphenyl)methyl-3,3-dimethyl-4-methylen-piperidin (**2a**) werden in 20 mL Aceton gelöst und mit 1 g konz. Schwefelsäure versetzt. Die ausgefallenen Kristalle werden abgesaugt und in 6 ml Dichlormethan¹⁾^{,2)} suspendiert. Hierzu gibt man, unter Kühlung bei 10-20°C, 9 g (68 mmol) AlCl₃. Es entsteht eine klare Lösung, die anschließend 2 h gekocht wird (Innentemperatur 46 °C). Das rotbraune Reaktionsgemisch wird auf Raumtemperatur abgekühlt, mit 25 ml Dichlormethan verdünnt und auf ca 100 g Eis gegeben. Unter Kühlung, bei 20 - 25 °C tropft man 100 ml 20%ige NaOH hinzu, trennt dann die organische Phase ab und extrahiert die wässrige Phase mit 25 ml Dichlormethan. Die vereinigten organischen Extrakte werden über Magnesiumsulfat getrocknet und das Lösungsmittel im Vakuum abdestilliert. Den Rückstand nimmt man in 10 ml Methanol auf und versetzt mit 3,1 g L-(+)-Weinsäure³ in 2 ml H₂O. Man läßt 10 Min im Eisbad auskristallisieren, verdünnt mit ca. 40 ml Aceton und saugt ab.
Ausbeute: 6,5g (82,3%), Schmp.: 236 °C.

**Tabelle 1:**

| Salz | Lösungsmittel | AlCl₃ | Temp. | Zeit | Ausbeute |
|---|---|---|---|---|---|
| Cl | Chlorbenzol | 4,0 eq | 90°C | 15' | 58,0% |
| Cl | Chlorbenzol | 4,0 eq | 75-80°C | 2 h | 61,7 % |
| Cl | CH₂Cl₂ | 4,0 eq | 20-25°C | 48 h | 44,4 % |
| Cl | CH₂Cl₂ | 3,2 eq | 20-25°C | 64 h | 54,4 % |
| Cl | C₂H₄Cl₂ | 4,0 eq | 55-60°C | 6 h | 42,0 % |
| Cl | C₂H₄Cl₂ | 3,2 eq | 42°C | 5 h | 78,8 % |
| Br | Chlorbenzol | 4,0 eq | 60°C | 2 h | 63,6 % |
| SU1 | C₂H₄Cl₂ | 3,4 eq | 60-65°C | 2 h | 85,3 % |
| SU1 | C₂H₄Cl₂ | 3,4 eq | 55-60°C | 30' | 91,1 % |
| SU1 | C₂H₄Cl₂ | 3,4 eq | 50-55°C | 30' | 90,5 % |
| SU1 | CH₂Cl₂ | 3,4 eq | 55 °C Autoklav | 1,5 h | 82,0 % |
| SU1 | CH₂Cl₂ | 3,4 eq | 46-47°C Brei | 2 h | 90,3 % |
| SU1 | DMAA | 8,0 eq | 80-90°C | 3 h | 60 % |

¹ Die alternative Verwendung von 1,2-Dichlorethan, liefert nach inverser Zugabe von AlCl₃ und nach 30 Min. bei 55°C, 78 % Benzomorphan.
² Die Reaktion in Dichlormethan bei 55°C unter Druck, liefert nach 1,5 Std. das Benzomorphan in 82 % Ausbeute.
³ Alternativ kann zur Kristallisation 62%ige HBr benutzt werden. Man isoliert das entsprechende Hydrobromid in 77% Ausbeute.

### Beispiel 2: (-)-3'-Methoxy-5,9,9-trimethyl-6,7-benzomorphan-tartrat ((-)-5bTA)

8,6 g (35 mmol) (+)-2-(3-Methoxyphenyl)methyl-3,3-dimethyl-4-methylen-piperidin (**2b**) werden in 35 mL Aceton gelöst und mit 1,8 g konz. Schwefelsäure versetzt. Die ausgefallenen Kristalle werden abgesaugt und in 10,5 ml 1,2-Dichlorethan suspendiert. Hierzu gibt man, unter Kühlung bei 20-30°C, 16 g (120 mmol) AlCl₃. Die Mischung wird rasch auf 55-70 °C erhitzt. Nach 30 min. läßt man auf Raumtemperatur abkühlen, verdünnt mit 100 ml Dichlormethan und versetzt mit 200 g Eiswasser. Unter Kühlung, bei 20 - 25 °C tropft man 300 ml 20%ige NaOH hinzu, trennt dann die organische Phase ab und extrahiert die wässerige Phase mit 150 ml Dichlormethan. Die vereinigten organischen Extrakte werden über Magnesiumsulfat getrocknet und das Lösungsmittel im Vakuum abdestilliert. Den Rückstand nimmt man in 20 ml Methanol auf und versetzt mit 5,4 g L-(+)-Weinsäure in 3 ml H₂O. Man läßt 10 Min im Eisbad auskristallisieren, verdünnt mit ca. 40 ml Aceton und saugt ab. Ausbeute: 10,9 g (79%), Schmp.: 186 °C.

## Patentansprüche

1. Verfahren zur Herstellung von R- bzw. S-Norbenzomorphanen der allgemeinen Formel I
worin 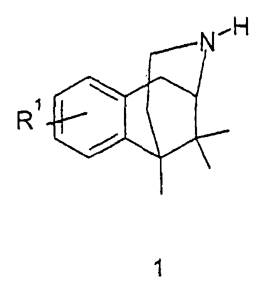 R¹ = H, C₁-C₈-Alkyl, C₁-C₈-Alkoxy, Hydroxy, Halogen
bedeuten kann, **dadurch gekennzeichnet, daß** man ein 4-Methylenpiperidinderivat der allgemeinen Formel 2 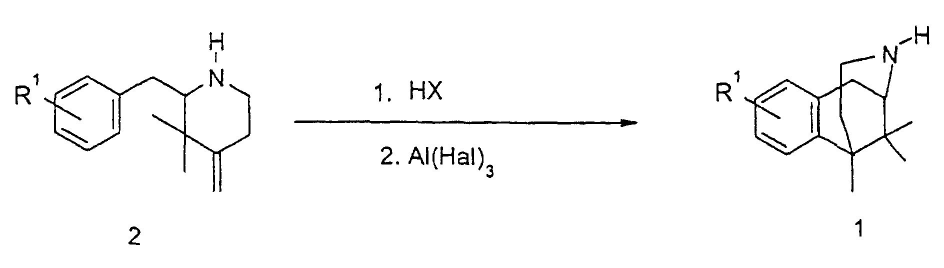 mit einer Säure in das entsprechende Säureaddditionssalz überführt
und das Salz in einem Reaktionsmedium mit einem Aluminium(III) halogenid, bevorzugt Aluminiumtribromid oder Alumniumtrichlorid, bei einer Temperatur in einem Intervall von 0 bis 150 °C umsetzt und das Reaktionsprodukt nach erfolgter Umsetzung isoliert.

2. Verfahren nach Anspruch 1, **dadurch gekennzeichnet, daß** man als Reaktionsmedium einen halogenierten aliphatischen oder aromatischen Kohlenwasserstoff oder ein Säureamid oder Mischungen der genannten Lösungsmittel einsetzt.

3. Verfahren nach Anspruch 2, **dadurch gekennzeichnet, daß** man als Reaktionsmedium ein mono- oder polychloriertes Alkan mit 1 bis 3 C-Atomen, ein chloriertes Benzol oder Benzolderivat oder ein Amid einer Carbonsäure mit 1 bis 3 C-Atomen im Carboxylrest oder Mischungen der genannten Lösungsmittel einsetzt.

4. Verfahren nach Anspruch 3, **dadurch gekennzeichnet, daß** man als Reaktionsmedium Dichlormethan, 1,2-Dichlorethan, Chlorbenzol oder Dimethylacetamid oder Mischungen der genannten Lösungsmittel einsetzt.

5. Verfahren nach einem der Ansprüche 1 bis 4, **dadurch gekennzeichnet, daß** man die Reaktion in einem Temperaturintervall von 20 bis 150 °C durchführt.

6. Verfahren nach Anspruch 5, **dadurch gekennzeichnet, daß** man die Reaktion in einem Temperaturintervall von 40 bis 70 °C durchführt.

7. Verfahren nach einem der Ansprüche 1 bis 6, **dadurch gekennzeichnet, daß** man 2 bis 12 Äquivalente Aluminium(III)halogenid bezogen auf das Edukt einsetzt.

8. Verfahren nach Anspruch 7, **dadurch gekennzeichnet, daß** man 3 bis 10 Äquivalente Aluminium(III)halogenid bezogen auf das Edukt einsetzt.

9. Verfahren nach Anspruch 8, **dadurch gekennzeichnet, daß** man 3 bis 5 Äquivalente Aluminium(III)bromid oder Aluminium(III)chlorid bezogen auf das Edukt einsetzt.

10. Verfahren nach einem der Ansprüche 1 bis 9, **dadurch gekennzeichnet, daß** man als Piperidinderivat (+)-2-(3-Methoxyphenyl)methyl-3,3-dimethyl-4-methylen-piperidin einsetzt.

11. Verfahren nach einem der Ansprüche 1 bis 9, **dadurch gekennzeichnet, daß** man als Ausgangsbase (-)-2-(3-Methoxyphenyl)methyl-3,3-dimethyl-4-methylen-piperidin einsetzt.

12. Verfahren nach einem der Ansprüche 1 bis 10,**dadurch gekennzeichnet, daß** man das Piperidinderivat in Form eines Additionssalzes mit einer Mineralsäure einsetzt.

13. Verfahren nach Anspruch 12, **dadurch gekennzeichnet, daß** man das Piperidinderivat in Form eines Additionssalzes mit einer Halogenwasserfstoffäure oder Schwefelsäure einsetzt.

14. Verfahren nach Anspruch 13, **dadurch gekennzeichnet, daß** man das Piperidinderivat in Form eines Additionssalzes mit Salzsäure, Bromwasserstoffäure oder Schwefelsäure einsetzt.

## Claims

1. Process for preparing R- or S-norbenzomorphanes of general formula 1 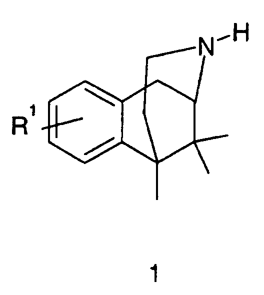 wherein
R¹ may denote H, C₁₋₈-alkyl, C₁₋₈-alkoxy, hydroxy or halogen, **characterised in that** a 4-methylene-piperidine derivative of general formula 2 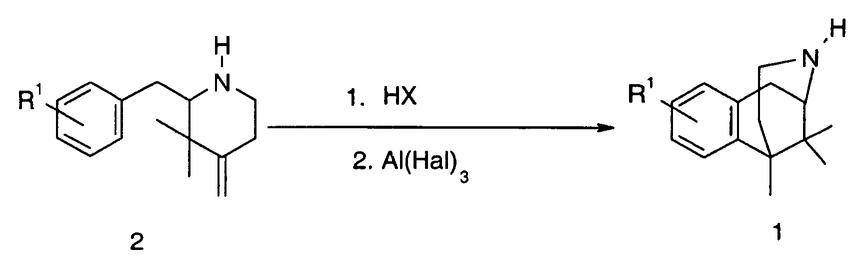 is converted with an acid into the corresponding acid addition salt
and the salt is reacted in a reaction medium with an aluminium (III) halide, preferably aluminium tribromide or aluminium trichloride, at a temperature in the range from 0 to 150°C and after the reaction is complete the reaction product is isolated.

2. Process according to claim 1, **characterised in that** the reaction medium used is a halogenated aliphatic or aromatic hydrocarbon or an acid amide or mixtures of the aforementioned solvents.

3. Process according to claim 2, **characterised in that** the reaction medium used is a mono- or polychlorinated alkane having 1 to 3 carbon atoms, a chlorinated benzene or benzene derivative or an amide of a carboxylic acid having 1 to 3 carbon atoms in the carboxyl group or mixtures of the abovementioned solvents.

4. Process according to claim 3, **characterised in that** the reaction medium used is dichloromethane, 1,2-dichloroethane, chlorobenzene or dimethylacetamide or mixtures of the aforementioned solvents.

5. Process according to one of claims 1 to 4, **characterised in that** the reaction is carried out in a temperature range of from 20 to 150°C.

6. Process according to claim 5, **characterised in that** the reaction is carried out in a temperature range of from 40 to 70°C.

7. Process according to one of claims 1 to 6, **characterised in that** 2 to 12 equivalents of aluminium (III) halide are used, based on the educt.

8. Process according to claim 7, **characterised in that** 3 to 10 equivalents of aluminium (III) halide are used, based on the educt.

9. Process according to claim 8, **characterised in that** 3 to 5 equivalents of aluminium (III) bromide or aluminium (III) chloride are used, based on the educt.

10. Process according to one of claims 1 to 9, **characterised in that** (+)-2-(3-methoxyphenyl)methyl-3,3-dimethyl-4-methylene-piperidine is used as the piperidine derivative.

11. Process according to one of claims 1 to 9, **characterised in that** (-)-2-(3-methoxyphenyl)methyl-3,3-dimethyl-4-methylene-piperidine is used as the starting base.

12. Process according to one of claims 1 to 10, **characterised in that** the piperidine derivative is used in the form of an addition salt with a mineral acid.

13. Process according to claim 12, **characterised in that** the piperidine derivative is used in the form of an addition salt with a hydrohalic acid or sulphuric acid.

14. Process according to claim 13, **characterised in that** the piperidine derivative is used in the form of an addition salt with hydrochloric, hydrobromic or sulphuric acid.

## Revendications

1. Procédé de production de R- ou S-norbenzomorphanes de formule générale I 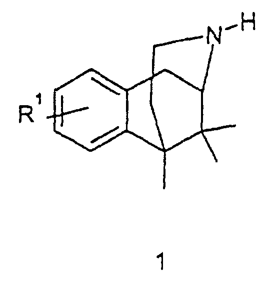 où
R¹ peut représenter H, alkyle en C₁₋₈, alcoxy en C₁₋₈, hydroxyle, halogène, **caractérisé en ce que** l'on convertit un dérivé de 4-méthylènepipéridine de formule générale 2 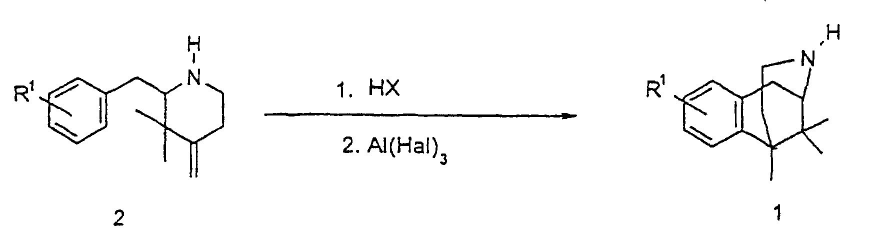 avec un acide en le sel d'addition d'acide correspondant,
et on fait réagir le sel dans un milieu réactionnel avec un halogénure d'aluminium (III), de préférence de tribromure d'aluminium ou le trichlorure d'aluminium, à une température dans un intervalle de 0 à 150°C et on isole le produit réactionnel après la fin de la réaction.

2. Procédé selon la revendication 1 **caractérisé en ce que** l'on utilise comme milieu réactionnel un hydrocarbure aliphatique ou aromatique halogéné ou un amide d'acide on des mélanges des solvants cités.

3. Procédé selon la revendication 2 **caractérisé en ce que** l'on utilise comme milieu réactionnel un alcane mono- ou polychloré ayant 1 à 3 atomes de carbone, un benzène ou dérivé benzénique chloré ou un amide d'un acide carboxylique ayant 1 à 3 atomes de carbone dans le reste carboxyle ou des mélanges des solvants cités.

4. Procédé selon la revendication 3 **caractérisé en ce que** l'on utilise comme milieu réactionnel le dichlorométhane, le 1,2-dichloroéthane, le chlorobenzène ou le diméthylacétamide ou des mélanges des solvants cités.

5. Procédé selon l'une des revendications 1 à 4 **caractérisé en ce que** l'on conduit la réaction dans un intervalle de température de 20 à 150°C.

6. Procédé selon la revendication 5 **caractérisé en ce que** l'on conduit la réaction dans un intervalle de température de 40 à 70°C.

7. Procédé selon l'une des revendications 1 à 6 **caractérisé en ce que** l'on utilise 2 à 12 équivalents d'halogénure d'aluminium (III) par rapport au produit de départ.

8. Procédé selon la revendication 7 **caractérisé en ce que** l'on utilise 3 à 10 équivalents d'halogénure d'aluminium (III) par rapport au produit de départ.

9. Procédé selon la revendication 8 **caractérisé en ce que** l'on utilise 3 à 5 équivalents de bromure d'aluminium (III) ou de chlorure d'aluminium (III) par rapport au produit de départ.

10. Procédé selon l'une des revendications 1 à 9 **caractérisé en ce que** l'on utilise comme dérivé de pipéridine la (+)-2-(3-méthoxyphényl)méthyl-3,3-diméthyl-4-méthylène-pipéridine.

11. Procédé selon l'une des revendications 1 à 9 **caractérisé en ce que** l'on utilise comme base de départ la (-)-2-(3-méthoxyphényl)méthyl-3,3-diméthyl-4-méthylène-pipéridine.

12. Procédé selon l'une des revendications 1 à 10 **caractérisé en ce que** l'on utilise le dérivé de pipéridine sous forme d'un sel d'addition avec un acide minéral.

13. Procédé selon la revendication 12 **caractérisé en ce que** l'on utilise le dérivé de pipéridine sous forme d'un sel d'addition avec un acide halogénhydrique ou l'acide sulfurique.

14. Procédé selon la revendication 13 **caractérisé en ce que** l'on utilise le dérivé de pipéridine sous forme d'un sel d'addition d'acide avec l'acide chlorhydrique, l'acide bromhydrique ou l'acide sulfurique.
